# EUROPÄISCHE PATENTSCHRIFT

(11) **EP 0 422 520 B1**
(45) Veröffentlichungstag und Bekanntmachung des Hinweises auf die Patenterteilung: **25.01.1995**
(21) Anmeldenummer: 90119148.6
(22) Anmeldetag: 05.10.1990
(51) Int. Cl.: C07C 51/58, C07C 53/48

(54) **Verfahren zur Herstellung von Dichlor- oder Trichloracetylchlorid**
Process for preparing dichloro- or trichloro acetyl chloride
Procédé de préparation de chlorure de dichloro- ou trichloro acétyle

(30) Priorität: 07.10.1989 DE 3933559
(43) Veröffentlichungstag der Anmeldung: 17.04.1991
(73) Patentinhaber: HOECHST AKTIENGESELLSCHAFT, 65926 Frankfurt am Main (DE)
(72) Erfinder: Freyer, Walter, Dr., W-8901 Leitershofen (DE); Miltenberger, Karlheinz, Dr., W-8906 Gersthofen (DE); Schmidt, Manfred, W-8870 Günzburg (DE)

(56) Entgegenhaltungen:
- CH-A- 324 906
- DE-C- 759 963
- US-A- 2 736 695

## Beschreibung

Die Erfindung bezieht sich auf ein schonendes Verfahren zur Herstellung von Dichloracetylchlorid oder Trichloracetylchlorid.

Bekanntermaßen werden Dichlor- und Trichloracetylchlorid durch Einwirkung von Sauerstoff oder sauerstoffhaltiger Gase, wie beispielsweise Luft, auf Trichlorethan oder Tetrachlorethan erhalten. Gewöhnlich werden diese Umsetzungen bei einer Temperatur zwischen Raumtemperatur und 200°C und drucklos oder unter Druck durchgeführt und durch Radikalbildner oder Bestrahlung mit kurzwelligem Licht gestartet und in Gang gehalten. Dabei entstehen neben den Säurechloriden und den entsprechenden Epoxiden auch geringe Mengen höhersiedender Säurechloride sowie gasförmige Nebenprodukte, wie beispielsweise Chlorwasserstoff, Kohlenmonoxid und Phosgen.
Das gebildete Epoxid kann durch Zugabe von Stickstoffbasen zum Säurechlorid umgelagert werden (vgl. DE 2 533 181, DE 2 050 562, DE 1 568 547).

Der Nachteil der bekannten Verfahren besteht darin, daß die Reaktionszeiten zu lang sind. Bei einem herkömmlichen Oxidationsansatz mit 4000 kg Trichlorethen, der ca. 60 h benötigt, damit 99,7 % des Trichlorethens oxidiert sind, haben sich bereits nach ca. 30 h ca. 80 % des Trichlorethens in Dichloracetylchlorid bzw. Trichlorethenepoxid umgewandelt. Zur Oxidation der restlichen 20 % werden nochmals 30 h benötigt. Durch diese langanhaltende Oxidation wird Dichloracetylchlorid zu unerwünschten Nebenprodukten, wie beispielsweise Phosgen, abgebaut.

Es bestand daher die Aufgabe, ein Verfahren zur Herstellung von Dichloracetylchlorid oder Trichloracetylchlorid zu finden, welches in kürzerer Zeit durchführbar ist.

Gefunden wurde, daß die Aufgabe lösbar ist, wenn die Oxidation des Trichlorethens oder Tetrachlorethens in dünner Schicht vorgenommen wird.

Die Erfindung betrifft somit ein Verfahren zur kontinuierlichen Herstellung von Dichlor- oder Trichloracetylchlorid durch Umsetzung von Trichlorethen oder Tetrachlorethen mit Sauerstoff in flüssiger Phase und unter Bestrahlung mit kurzwelligem Licht bei einer Temperatur von 70 bis 140°C und einem Druck von 1 bis 20 bar, dadurch gekennzeichnet, daß man das Ausgangsprodukt in einem dünnen Film durch die Reaktionszone führt, das Abgas periodisch abläßt mit einer solchen Häufigkeit, daß die Druckschwankungen in der Apparatur nicht größer als etwa 10 % sind, und den Sauerstoff unter im wesentlichen statischen Druck hält.

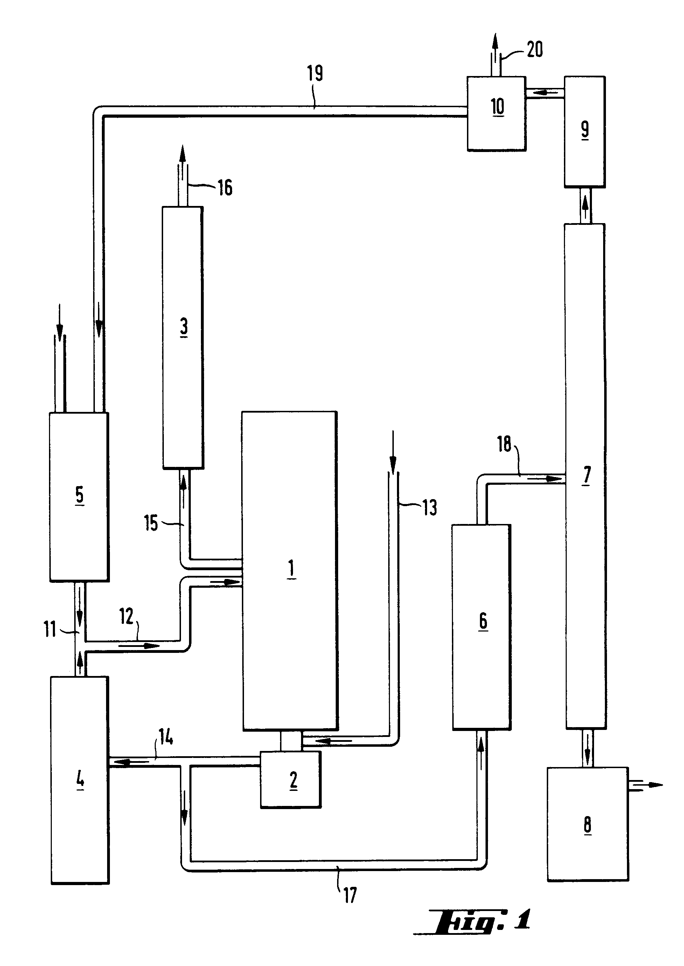

Das erfindungsgemäße Verfahren wird in einer Anlage durchgeführt, welche gemäß Figur 1 aus einem Fallfilm-Photoreaktor (1) mit Umwälzpumpe (2), einem Abgaskühler (3), einem Puffergefäß (4) und einem Vorratsgefäß (5) besteht. Wenn Trichlorethen in Dichloracetylchlorid umgewandelt werden soll, kommt für die Abtrennung des Säurechlorids noch ein Vorwärmer (6), eine Destillationskolonne (7), eine Vorlage (8) für DAC, ein Rückflußkühler (9) und eine Vorlage (10) für Trichlorethen dazu.

Trichlorethen oder Tetrachlorethen werden der Anlage über das Vorratsgefäß (5) zugeführt. Von dort aus gelangt das Chlorolefin über die Leitungen (11) und (12) in den Fallfilm-Photoreaktor (1), wo es als dünner Film durch die Reaktionszone geführt und dort unter Belichtung durch eine UV-Lichtquelle mit Sauerstoff umgesetzt wird, welcher durch die Leitung (13) zugeführt wird. Das Reaktionsgemisch sammelt sich in der Umwälzpumpe (2) und wird von ihr in das Puffergefäß (4) gefördert, von wo aus es durch Leitung (12) wieder in den Reaktor (1) gelangen kann. Bei der Reaktion entstehende Gase entweichen durch Leitung (15) und werden im Abgaskühler (3) von mitgerissener Flüssigkeit getrennt. Über Leitung (16) gelangen sie zur Abgasreinigung.

Bei der Herstellung von Dichloracetylchlorid wird durch Leitung (17) ein Teil des Reaktionsgemisches abgezweigt, im Vorwärmer (6) erhitzt und durch Leitung (18) der Destillationskolonne (7) zugeführt. Das höhersiedende Dichloracetylchlorid sammelt sich in Vorlage (8), das leichtersiedende Trichlorethen wird im Kühler (9) kondensiert und sammelt sich in Vorlage (10). Von hier aus läuft das zurückgewonnene Trichlorethen durch Leitung (19) in das Vorratsgefäß (5), während die Abgase die Anlage durch Leitung (20) verlassen.

Im Fallfilm-Photoreaktor fließt das Chlorolefin oder die im Kreislauf geführte Reaktionsmischung als dünner Film auf der Innenseite eines zylindrischen Rohres und wird von der Außenseite dieses Rohres temperiert. Im Inneren des Rohres befinden sich in einem Schutzrohr - ohne Berührung mit den Reaktanten - eine oder mehrere UV-Lichtquellen. Die Reaktionstemperatur beträgt 70 bis 140, vorzugsweise 70 bis 110°C.

Die Reaktion wird unter einem Sauerstoffdruck von 1 bis 20, vorzugsweise 1 bis 4 bar, durchgeführt. Dabei wird der Reaktor nicht kontinuierlich vom Sauerstoff durchströmt, sondern das Abgas wird periodisch abgelassen mit einer solchen Häufigkeit, daß die Druckschwankungen in der Apparatur nicht größer als etwa 10 % sind. Dabei wird der mit dem Abgas entweichende Sauerstoff ersetzt.

Die Umlagerung der bei der Oxidation ebenfalls gebildeten Epoxide in die Säurechloride in Gegenwart von organischen Stickstoffbasen oder deren Salzen erfolgt außerhalb der Oxidationszone.

Zur Verhütung von Nebenreaktionen, beispielsweise durch Weiteroxidation des gebildeten Säurechlorids oder Epoxids, wird das Reaktionsgemisch im Kreis geführt und durch Ausschleusen von stündlich 10 bis 20 % und durch Zugabe von frischem Trichlorethen oder Tetrachlorethen ein konstanter Säurechloridgehalt im Reaktionsgemisch eingestellt. Dieser Gehalt beträgt 40 bis 80, vorzugsweise 60 bis 70 % Säurechlorid, bezogen auf das Reaktionsgemisch. Bei der kontinuierlichen Herstellung von Dichloracetylchlorid wird der ausgeschleuste Anteil des Reaktionsgemisches einer kontinuierlichen Destillation zugeführt. Im Blasensumpf sammelt sich die höhersiedende Komponente, hier das Dichloracetylchlorid 98 bis 99%ig, an und die niedrigsiedenden Komponenten, auch das Trichlorethen, werden über Kopf abdestilliert. Das bei der Destillation abgetrennte Trichlorethen wird mit frischem Trichlorethen versetzt und dem Oxidationskreislauf wieder zugeführt.

In analoger Weise wird Tetrachlorethen zu Trichloracetylchlorid oxidiert. Wegen des nur um 2°C höheren Siedepunktes des Tetrachlorethens gegenüber dem Trichloracetylchlorid ist eine kontinuierliche destillative Trennung nicht möglich. Es empfiehlt sich daher gegebenenfalls auf die Reinigung des Trichloracetylchlorids zu verzichten und seine direkte Weiterverarbeitung (z.B. Veresterung) anzuschließen.

Durch das erfindungsgemäße Verfahren wird die Reaktionszeit wirkungsvoll verkürzt und der Anfall von Nebenprodukten verringert.

Die nachfolgenden Beispiele sollen die Erfindung näher erläutern.

### Beispiel 1

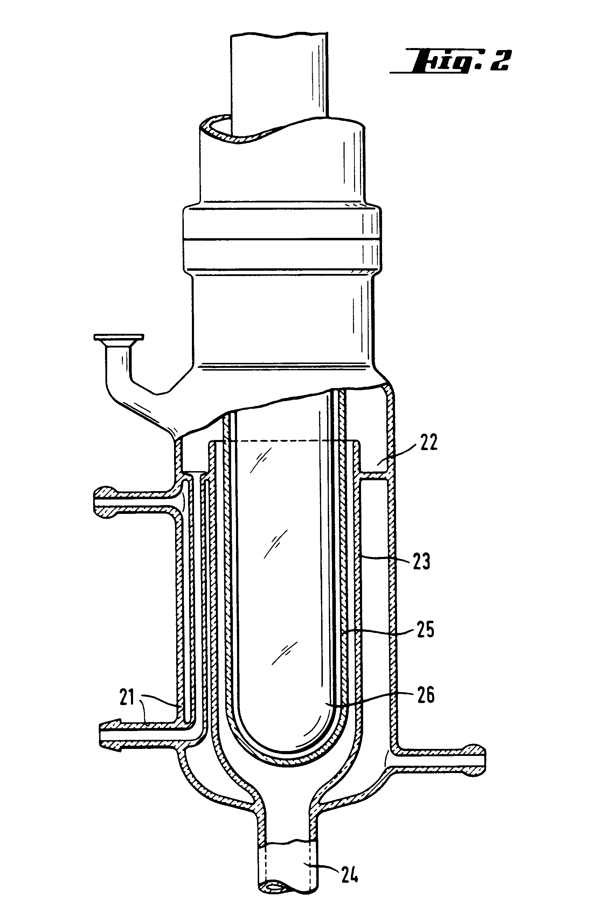

Die Versuche wurden in einem Fallfilm-Photoreaktor nach Prof. de Meijere durchgeführt. Die wesentlichen Merkmale dieses Reaktors sind in Figur 2 dargestellt. Das Reaktionsgemisch tritt durch das Rohr (21) in den Reaktor ein, füllt die Kreisrinne (22) und fließt auf der Innenseite des zylindrischen Rohres (23) als dünner Film durch den unteren Stutzen (24) zur nicht gezeigten Umwälzpumpe. In dem Schutzrohr (25) steckt eine UV-Lichtquelle (26) mit Kühlmantel. Derartige Reaktoren sind im Fachhandel käuflich erhältlich.
Der Reaktor war mit einem Vorratsgefäß für das Reaktionsgemisch - in dem das Epoxid mit Pyridin umgelagert wurde -, einem Tropftrichter für das Ausgangsmaterial und einem Intensivkühler ausgestattet. Der Intensivkühler war mit einer Quecksilbertauchung zur Einstellung des Druckes versehen. Als UV-Lichtquelle wurde ein Quecksilber-Hochdruckbrenner TQ 150, der ebenfalls im Handel erhältlich ist, verwendet. Der Quecksilber-Hochdruckbrenner steckte in einem Tauchrohr aus Quarz, er wurde mit einem Stickstoffgasstrom gekühlt. Für die Versuche wurde jeweils das Ausgangsmaterial in die Apparatur gegeben, die Umwälzpumpe eingeschaltet und die Apparatur über den Thermostat aufgeheizt. Nach Erreichen der Reaktionstemperatur wurde der UV-Brenner gezündet und Sauerstoff im Gegenstrom mittels einer Fritte über den Film geleitet. Im drucklosen Zustand wurde der Sauerstoffstrom, der mit HCl, CO und Phosgen beladen war, direkt über den Intensivkühler abgeleitet und entsorgt. Bei Arbeiten unter Druck war dem Intensivkühler eine Quecksilbertauchung nachgeschaltet, mit der Sauerstoffdrucke von 0,5 und 0,75 bar eingestellt werden konnten. Zur Regenerierung des Sauerstoffs wurde der Überdruck an der Gasflasche so eingestellt, daß es zu geringem Abblasen am Intensivkühler kam.

Zu Beginn wurde das Reaktionsgemisch bis zu einem gewissen Säurechloridgehalt im Kreise geführt.

Im kontinuierlichen Betrieb wurde im Falle der DAC-Herstellung ein Teil des Reaktionsgemisches dem Kreislauf entnommen und einer kontinuierlichen Destillation zugeführt.

1612 g Trichlorethen wurden mit 90 mg Pyridin (0,006 %) versetzt und bei einer Temperatur von 80°C und unter einem Gesamtdruck von 1,5 bar oxidiert. Die Abnahme des Trichlorethens und die Zunahme der Reaktionsprodukte wurde gaschromatographisch verfolgt. Nach ca. 6,5 h lag der Trichlorethen-Anteil bei 0,4 %. Die Anteile an Dichloracetylchlorid und Trichlorethylenoxid stiegen zunächst linear an, um nach ca. 5 h immer weniger zuzunehmen und einem Grenzwert zuzustreben. Die Gesamtausbeute an DAC und Epoxid betrug 93 %. Die Ergebnisse der gaschromatographischen Analyse sind in der Tabelle 1 zusammengefaßt:

**Tabelle 1**

| Reaktionszeit (h) | Trichlorethen (%) | DAC (%) | Epoxid (%) |
|---|---|---|---|
| 1 | 82 | 14 | 4 |
| 2 | 61 | 28 | 6 |
| 3 | 42 | 42 | 9 |
| 4 | 23 | 57 | 12 |
| 5 | 9,5 | 70 | 15 |
| 6 | 2 | 80 | 11 |
| 7 | - | 86 | 7 |

### Beispiele 2 bis 4

Jeweils 1612 g Trichlorethen wurden mit 90 mg Pyridin (0,006 %) versetzt und bei 73°C unter verschiedenen Drucken wie im Beispiel 1 im Fallfilm-Photoreaktor oxidiert. Erwartungsgemäß stieg mit höherem Druck auch die Reaktionsgeschwindigkeit an. Der DAC-Gehalt stieg zunächst bis zu einem DAC-Gehalt von ca. 70 % linear an. Danach wurde der Zuwachs geringer und der Gehalt näherte sich einem oberen Grenzwert, der bei allen Drucken bei ca. 80-85 % lag.

Die Ergebnisse der gaschromatographischen Analyse zeigt Tabelle 2:

**Tabelle 2**

| Reaktionszeit (h) | DAC-Anteil in % bei 73°C und verschiedenen Drucken | | |
|---|---|---|---|
| | 1 bar | 1,5 bar | 1,75 bar |
| 1 | 11 | 15 | 18 |
| 2 | 22 | 29 | 34 |
| 3 | 33 | 42 | 51 |
| 4 | 45 | 57 | 68 |
| 5 | 55 | 70 | 80 |
| 6 | 66 | 79 | 85 |
| 7 | 77 | 84 | 86 |
| 8 | 84 | 86 | - |
| 9 | 86 | - | - |

### Beispiel 5

3500 g Trichlorethen wurden kontinuierlich im Fallfilm-Photoreaktor gemäß Beispiel 1 bis zu einem DAC-Gehalt von 65 % oxidiert. Das Epoxid wurde kontinuierlich durch anschließende Zugabe von 200 mg Pyridin (0,006 %) umgelagert. Ein Teil des Reaktionsgemisches wurde ausgeschleust, durch frisches Trichlorethen ersetzt und einer kontinuierlichen Destillation zugeführt. Unter Berücksichtigung des in der Apparatur verbleibenden Anteils und des zurückgeführten Trichlorethens wurde eine Ausbeute an DAC von 95 % erzielt.

### Beispiel 6

Analog dem Beispiel 1 wurden 1631 g Tetrachlorethen bei 110°C und 1,75 bar Druck im Fallfilm-Photoreaktor oxidiert.

Die TAC-Zunahme wurde gaschromatographisch und die Tetrachlorethen-Abnahme IR-spektrographisch verfolgt. Die Oxidation wurde solange weitergeführt, bis keine TAC-Zunahme mehr erfolgte. Dabei verblieb im TAC ein Restgehalt von 3 % Tetrachlorethen, der durch Weiteroxidation nicht mehr unterschritten wurde.

Die Ergebnisse der gaschromatographischen Analyse der Oxidation zeigt Tabelle 3:

**Tabelle 3**

| Reaktionszeit (h) | Tetrachlorethen (%) | TAC (%) |
|---|---|---|
| 1 | 80 | 25 |
| 2 | 62 | 45 |
| 3 | 46 | 60 |
| 4 | 32 | 72 |
| 5 | 20 | 82 |
| 6 | 9 | 91 |
| 7 | 3 | - |

Wegen des geringen Unterschiedes zwischen den Siedepunkten des Tetrachlorethens und des Trichloracetylchlorids ist eine Trennung durch Destillation nur unter sehr großem Aufwand möglich. Das rohe Säurechlorid läßt sich jedoch direkt weiterverarbeiten, beispielsweise zu den Estern, welche sich leichter vom Tetrachlorethen trennen lassen.

### Vergleichsversuch

In einem Vierhalskolben, der mit Rührer, Thermometer, Rückflußkühler und Gaseinleitungsrohr ausgestattet war, wurden 1612 g Trichlorethen, enthaltend 90 mg Pyridin, unter Bestrahlung mit UV-Licht und Durchleiten von Sauerstoff (Normaldruck) bei 73°C oxidiert.

Die Umsetzung wurde gaschromatographisch verfolgt:

| Reaktionszeit (h) | Trichlorethen (%) | DAC (%) | Epoxid (%) |
|---|---|---|---|
| 17 | 1,0 | 70,9 | 27,0 |
| 18,5 | 0,04 | 73,1 | 25,6 |

## Patentansprüche

1. Verfahren zur kontinuierlichen Herstellung von Dichlor- oder Trichloracetylchlorid durch Umsetzung von Trichlorethen oder Tetrachlorethen mit Sauerstoff in flüssiger Phase und unter Bestrahlung mit kurzwelligem Licht bei einer Temperatur von 70 bis 140°C und einem Druck von 1 bis 20 bar, dadurch gekennzeichnet, daß man das Ausgangsprodukt in einem dünnen Film durch die Reaktionszone führt, das Abgas periodisch abläßt mit einer solchen Häufigkeit, daß die Druckschwankungen in der Apparatur nicht größer als etwa 10 % sind,und den Sauerstoff unter im wesentlichen statischen Druck hält.

2. Verfahren nach Anspruch 1, dadurch gekennzeichnet, daß zur kontinuierlichen Herstellung von Dichloracetylchlorid ein Gehalt an Säurechlorid im Reaktionsgemisch im Bereich von 40 bis 80 Gew.-%, bezogen auf die Gesamtmenge, eingehalten wird, in dem stündlich 10 bis 20 % des Reaktionsgemisches ausgeschleust und durch fraktionierte Destillation aufgearbeitet werden und das Reaktionsgemisch durch frisches Trichlorethen ergänzt wird.

## Claims

1. A process for the continuous preparation of dichloro- or trichloroacetyl chloride by reaction of trichloroethene or tetrachloroethene with oxygen in the liquid phase under irradiation with light of short wavelength at a temperature of 70 to 140°C under a pressure of 1 to 20 bar, which comprises passing the starting substance in a thin film through the reaction zone, letting off the waste gas periodically at a frequency such that the pressure variations in the apparatus do not exceed about 10% and keeping the oxygen essentially under static pressure.

2. The process as claimed in claim 1, wherein, for continuous preparation of dichloroacetyl chloride, a content of acid chloride in the reaction mixture in the range from 40 to 80% by weight, based on the total amount, is maintained by removing 10 to 20% of the reaction mixture per hour, and working this up by fractional distillation and topping up the reaction mixture with fresh trichloroethene.

## Revendications

1. Procédé de préparation en continu de chlorures de dichloro- ou trichloroacétyle par réaction du trichloréthène ou du tétrachloréthène avec de l'oxygène en phase liquide sous irradiation de courtes longueurs d'onde, à une température de 70 à 140°C et sous une pression de 1 à 20 bar, **caractérisé** en ce que l'on fait passer le produit de départ sous la forme d'un film mince au travers de la zone de réaction, on soutire périodiquement le gaz résiduel selon une fréquence telle que la variation de pression à l'intérieur de l'appareillage ne soit pas supérieure à environ 10 % et que l'oxygène soit maintenu sous une pression essentiellement statique.

2. Procédé selon la revendication 1, **caractérisé** en ce que, lors de la préparation en continu du chlorure de dichloroacétyle, la teneur en chlorure d'acide dans le mélange réactionnel est maintenue dans une zone de 40 à 80 % en poids, rapporté à la quantité totale, 10 à 20 % du mélange réactionnel est prélevé par heure et est traité par une distillation fractionnée, et le mélange réactionnel est complété par du trichloréthène frais.
